# EUROPEAN PATENT APPLICATION

(11) **EP 1 674 099 A1**
(43) Date of publication of application: **28.06.2006**
(21) Application number: 06003150.7
(22) Date of filing: 17.12.2001
(51) Int. Cl.: A61K 31/44, A61K 31/55, A61P 9/00, A61P 9/02, A61P 9/04, A61P 9/10, A61P 9/12, A61P 11/00, A61P 27/00, A61P 43/00

(54) **Therapeutic combination of amlodipine and benazepril / benazeprilat**

(30) Priority: 18.12.2000 WO PCT/US00/34246
(62) Divisional of application: 01992150.1
(71) Applicant: Novartis AG, 4056 Basel (CH)
(72) Inventor: Daley, William, Lionel, Morris Plains, NJ 07950 (US); Webb, Randy, Lee, Flemington, NJ 08822 (US); Comfort, Ann, Reese, New City, New York 10956 (US); Fleres, Santo, Joseph, Hillsborough, NJ 08844 (US); Royce, Alan, Edward, Saylorsburg, PA 18353 (US); Wei, William, Shifeng, Belle Mead, NJ 08502 (US)
(74) Representative: Roth, Peter Richard

(57) **Abstract**

The present invention especially relates to the use of a combination comprising
(1) an ACEI selected from the group consisting of benazepril, benazeprilat, and pharmaceutically acceptable salts thereof, and
(2) amlodipine or pharmaceutically acceptable salt thereof,
for the manufacture a medicament for the treatment or prevention or delay of progression of a condition selected from the group consisting of hypertension, congestive heart failure, angina, myocardial infarction, artherosclerosis, diabetic nephropathy, diabetic cardiac myopathy, renal insufficiency, peripheral vascular disease, left ventricular hypertrophy, cognitive dysfunction, blood pressure-related cerebrovasular disease, stroke, pulmonary disease or pulmonary hypertension and headache;
wherein
(i) the amount of amlodipine or a pharmaceutically acceptable salt thereof corresponds to 6 mg to 40 mg of the free base and
(ii) the amount of the ACE inhibitor or a pharmaceutically thereof corresponds to 20 mg to 160 mg of benazepril hydrochloride.

## Description

Calcium channel blockers (CCBs) and angiotensin converting enzyme inhibitors (ACEIs) are widely used for the treatment of hypertension and related diseases and conditions. A representative of the class of CCBs is amlodipine, while a representative of the class of ACEIs is benazepril or benazeprilat.

Amlodipine is 2-[(2-aminoethoxy)methyt]-4-(2-chtorophenyt)-1,4-dihydro-6-methyt-3,5-pyridinedicarboxylic acid 3-ethyl 5-methyl ester. It is sold commercially in the form of its besylate salt under the trademark NORVASC® as an antihypertensive. Amlodipine may be administered in free or pharmaceutically aceptable salt form. Where amlodipine dosages are set forth herein, it is understood that the amounts are the amounts corresponding to amlodipine free base equivalents, irrespective of the salt form used, unless otherwise indicated.

It is known that a chronic anti-hypertensive therapy with amlodipine is often associated with side effects such as dose-limiting peripheral edema, especially ankle edema. The amlodipine induced ankle edema, for example, is believed to be due to a preferential dilation of the precapillary arterioles in the leg and a resultant efflux of fluid into the interstitial space. The upper limit of mono-therapy with amlodipine is 10 mg per day, and lower doses are preferred for chronic treatments. Higher dosage formulations than 10 mg per day are not approved by regulatory authorities or marketed, as in many susceptible individuals, side effects, such as those mentioned above, may limit efficacy and may ultimately result in discontinuation of the therapy. As used herein, a "high dose" or a "higher dose" of amlodipine refers to daily dosage amounts greater than 5 mgs amlodipine, preferably from 6-40 mgs, more preferably 7.5-20 mgs, for example, 7.5, 10, 15, or 20 mgs, more preferably at least 10 mgs, e.g., 10, 12.5, 15, or 20 mgs, most preferably 10 or 20 mgs. For administration every other day, dosages of 10-60mgs, preferably 20 to 40 mgs, for example 20, 30, or 40 mgs" especially 40 mgs, are preferred.

Benazepril is [S-(R*,R*)]-3-[[1-(ethoxycarbonyl)-3-phenylpropyl]amino]-2,3,4,5-tetrahydro-2-oxo-1H-1-benazepine-1-acetic acid. It is sold commercially in the form of the hydrochloride salt under the trademarks LOTENSIN® or CIBACEN® as an antihypertensive. Benazeprilat is the diacid form of benazepril formed by cleavage of the ester group, and is the active metabolite of benazepril. Benazepril and benazeprilat may be administered in free or pharmaceutically acceptable salt form. Where benazepril dosages are set forth herein, it is understood that the are the amounts are amounts of benazepril or benazeprilat corresponding to benazepril hydrochloride equivalents, irrespective of the actual salt form used, unless otherwise indicated.

The therapy using benazepril or a pharmaceutically acceptable salt thereof or benazeprilat ranges from 10 mg to 80 mg per day. However, ACEIs may only be moderately effective in non-Caucasian populations, especially in African Americans, and in patients where the renin angiotensin aldosterone system (RAAS) is already suppressed. As used herein, a "high dose" or a "higher dose" of benazepril" refers to dosage amounts corresponding to greater than 20 mg benazepril hydrochloride, preferably from 21 to 160 mgs, preferably 40 to 80 mgs, for example, 40 mg or 80 mgs. Dosage amounts of this drug may be also be given every other day, in combination with amlodipine on the same day or on altemate days on which the amlodipine is administered. In both cases, the amount of benazepril would be preferably kept constant.

Fixed dose combinations of amlodipine and benazepril are being marketed under the trade name Lotrel®. Corresponding amounts of the active ingredients are 2.5 mg of amlodipine and 10 mg of benazepril, 5 mg of amlodipine and 10 mg of benazepril, and 5 mg of amlodipine and 20 mg of benazepril, the amounts of amlodipine corresponding to the free base and the amounts of benazepril corresponding to the hydrochloride. As used herein, the term "Lotrel® combination" refers to these dosage combinations.

There is a clear need to provide a combination comprising a higher amount of the CCB and/or of the ACEI showing not only a greater blood pressure control, but also showing unexpected and even more beneficial advantages over lower dose combinations of amlodipine and benazepril. Such advantages comprise using such a combination in chronic treatment, e.g. avoiding side effects associated with high doses of amlodipine, in achieving further beneficial effects.

Surprisingly, administration of a high dose combination of amlodipine and benazepril exhibits even more beneficial advantages over the known low dose combinations that are being marketed.

The therapeutic combination of amlodipine and benazepril contemplated herein includes administration of these compounds such that the combination of amlodipine and benzapril may be administered every other day. Optionally, when the combination is administered every other day, benazepril alone may be administered on the alternate days. Suitably, patients are provided with dosage forms comprising (i) combinations of amlodipine with benazepril and (ii) dosage forms comprising benazepril as active ingredient or placebo, in which case the two dosage forms may be provided in a kit of parts as described below. For example, packages will comprise daily dosage amounts of appropriate active ingredients in a dispenser, blister pack or with other suitable packaging and instructions to ensure that the appropriate tablets are taken on the alternating days and otherwise ensure proper compliance with a prescribed dosage schedule. In a related embodiment, a high dose amlodipine may be altemated with lower dosage amounts of this drug on a daily basis in combination with daily administration of benazepril. Typically, the daily dosage level of the benazepril in this regime would remain constant.

The high dose combination of amlodipine and benazepril or benazeprilate, respectively, as disclosed herein provides some surprising beneficial effects with an overall lack of adverse effects, selected from (but are not limited to) e.g. (i) a greater blood pressure control (either or both of systolic and diastolic blood pressure), especially in patients who do not achieve blood pressure levels defined as normal or optimal according to the WHO guidelines of the management of hypertension; (ii) reduction, prevention, attenuation or delay of side effects associated with amlodipine, such as the dose-limiting formation of peripheral edema, e.g. ankle edema; (iii) reduction of afterload; (iv) end-organ protection, especially in case of the treatment of angina; and (v) reduction, prevention, attenuation or delay of risks or incidences of morbidity and mortality, especially of morbidity and mortality associated with atherosclerosis.

A preferred patient population for applying the combination according to the present invention is selected from (i) those patients who do not achieve blood pressure levels defined as normal or optimal according to the WHO guidelines of the management of hypertension of 1999 (cf. *J Hypertens* 1999, 17:151-183); (ii) those patients with angina who require greater control of angina or blood pressure or both; (iii) those patients with heart failure, especially congestive heart failure, who require greater control of blood pressure, and (iv) patients suffering from pulmonary disease or pulmonary hypertension.

These surprising and beneficial effects can be proven by carrying out appropriate clinical trials or experiments in conventional animal test models.

For example, in clinical trials when administering a combination according to the present invention, a positive dose response is demonstrated, reduced side effects are observed and a more pronounced blood pressure lowering effect is observed in patients with different categories of hypertension, such as severe hypertension, as compared to a Lotrel® combination. (According to the WHO, patients who have a systolic blood pressure (SBP) of ≥180 mm Hg or a diastolic blood pressure (DBP) of ≥110 mm Hg or patients who have both SBP of ≥180 mm Hg and DBP of ≥110 mm Hg have severe hypertension.) Likewise, patients with essential hypertension (MSBBP ≥95 mm Hg to s 115 mm Hg) who have received the combination according to the present invention have a greater responder rate than those patients who receive a Lotrel® combination. Especially beneficial is the treatment of patients with moderate to severe hypertension (moderate hypertension being characterized by a DBP of approximately 100 mm Hg according to said WHO definitions), most preferable is treatment of patients with severe hypertension (e.g. mean sitting diastolic blood pressure = MSDBP > 105 mm Hg at baseline).

Corresponding clinical trials can be carried out e.g. in a double-blind, randomized, placebo-controlled, forced-titration, parallel-group trial. For example, following a 2-week washout period and a 2-week single-blind placebo run-in period, patients are randomized to receive either a Lotrel ® combination (e.g. 5 mg of amlodipine corresponding of the free base and 20 mg of benazepril corresponding to the hydrochloride) or a combination according to the present invention (e.g. 10 mg of amlodipine corresponding of the free base and 20 mg of benazepril corresponding to the free base) for 6 weeks; patients receiving placebo remain on placebo for the entire 8 weeks. The efficacy, e.g. the change of blood pressure from baseline to the endpoint, is to be determined as well as the safety, e.g. the incidence of potential side effects.

The combination according to the present invention can be used for the treatment (acute and especially chronic treatment) or prevention or delay of progression of a condition selected from the group consisting of hypertension (whether of the malignant, essential, reno-vascular, diabetic, isolated systolic, or other secondary type), heart failure, such as congestive heart failure, angina (whether stable or unstable), myocardial infarction, atherosclerosis, diabetic nephropathy, diabetic cardiac myopathy, renal insufficiency, peripheral vascular disease, left ventricular dysfunction, such as left ventricular hypertrophy, cognitive dysfunction (such as Alzheimer's, etc.), blood pressure-related cerebrovasular disease, stroke, pulmonary disease or pulmonary hypertension and headache. It can be used, likewise, for the prevention, reduction, attenuation and delay of progression of side effects assoaated with high dose application of amlodipine; and for the protection of end-organs, induding the kidneys and the heart, for example protection against left ventricular hypertrophy, right ventricular hypertrophy, e.g. as associated with pulmonary hypertension, and the like.

The present invention relates to the use of a combination comprising
(1) an ACE inhibitor, selected from the group consisting of benazepril, benazeprilat, and pharmaceutically acceptable salts thereof, and
(2) amlodipine or pharmaceutically acceptable salt thereof,
   for the manufacture of a medicament for the treatment or prevention or delay of progression of a condition selected from the group consisting of hypertension, congestive heart failure, angina, myocardial infarction, atherosclerosis, diabetic nephropathy, diabetic cardiac myopathy, renal insufficiency, peripheral vascular disease, left ventricular hypertrophy, cognitive dysfunction, blood pressure-related cerebrovasular disease, stroke, pulmonary disease or pulmonary hypertension and headache;
wherein
(i) the amount of amlodipine or a pharmaceutically acceptable salt thereof is a high dose as defined above, e.g., corresponding to 6 mg to 40 mg of the free base and
(ii) the amount of the ACE inhibitor or a pharmaceutically acceptable salt thereof is a high dose as defined above, e.g., corresponding to 20 mg to 160 mg of benazepril hydrochloride.

The invention likewise relates to a composition, such as a pharmaceutical composition e.g. for the treatment or prevention or delay of progression of a condition selected from the group consisting of hypertension, congestive heart failure, angina, myocardial infarction, atherosclerosis, diabetic nephropathy, diabetic cardiac myopathy, renal insufficiency, peripheral vascular disease, left ventricular hypertrophy, cognitive dysfunction, blood pressure-related cerebrovasular disease, stroke, pulmonary hypertension, and headache; comprising
(1) an ACEI selected from the group consisting of benazepril, benazeprilat, and pharmaceutically acceptable salts thereof, and
(2) amlodipine or pharmaceutically acceptable salt thereof, and
(3) a pharmaceutically acceptable carrier,
wherein
(i) the amount of amlodipine or a pharmaceutically acceptable salt thereof is a high dose as defined above, e.g., corresponding to 6 mg to about 40 mg of the free base and
(ii) the amount of the ACE inhibitor or a pharmaceutically thereof is a high dose as defined above, e.g., corresponding to 20 mg to 160 mg of benazepril hydrochloride.

Fixed combinations of amlodipine and benazepril in accordance with the present invention thus include combinations of high dose amlodipine and high dose benazepril, in ranges as described above, and also combinations at higher dosages of amlodipine than currently approved and provided in Lotrel® combinations, for example combinations containing amounts corresponding to 10-60 mg amlodipine and 20-160 mgs benazepril, e.g., combintations corresponding to (i) 21-40 mgs amlodipine, preferably 10-40 mgs amlodipine free base, e.g., 10, 15 or 20 mgs, and (ii) 81-160 mgs benazepril hydrochloride, preferably 20-80 mgs benazepril hydrochloride, for example combinations corresponding to
10mg amolodipine free base and 20 mgs benazepril HCl,
10 mg amlodipine free base and 40 mgs benazepril HCl,
20 mgs amlodipine free base and 40 mgs benzapril HCl, and
20 mgs amlodipine free base and 80 mgs benazepril HCl.
The amolidpine is preferably in the form of amlodipine besylate. The benazepril is preferably in the form of benazepril hydrochloride.

As used herein, "pharmaceutically acceptable carrier" includes compounds well known to one of skill in the art and comprises excipients and auxiliaries which facilitate processing of the active compounds into preparations which can be used pharmaceutically. Further detaits on techniques for formulation and administration may be found in the latest edition of Remington's Pharmaceutical Sciences (Maack Publishing Co., Easton, PA).

The present invention likewise relates to a method of treatment or prevention or delay of progression of a condition selected from the group consisting of hypertension, congestive heart failure, angina, myocardial infarction, atherosclerosis, diabetic nephropathy, diabetic cardiac myopathy, renal insufficiency, peripheral vascular disease, left ventricular hypertrophy, cognitive dysfunction, blood pressure-related cerebrovasular disease, stroke, pulmonary disease or pulmonary hypertension and headache, comprising administering to a warm-blooded animal including man in need thereof an effective amount of a combination comprising
(1) an ACEI selected from the group consisting of benazepril, benazeprilat, and pharmaceutically acceptable salts thereof, and
(2) amlodipine or pharmaceutically acceptable salt thereof,
wherein
(i) the amount of amlodipine or a pharmaceutically acceptable salt thereof in a high dose as defined above, e.g., corresponding to 6 mg to about 40 mg free base and
(ii) the amount of the ACE inhibitor or a pharmaceutically acceptable salt thereof is a high does as defined above, e.g., corresponding to 20 mg to 160 mg benazepril hydrochloride.

The corresponding active ingredients or a pharmaceutically acceptable salt thereof may also be used in form of a solvate, such as a hydrate or including other solvents, used for crystallization according to conventional methods.

The compounds to be combined can be present as pharmaceutically acceptable salts. As these compounds have at least one basic center, they can form acid addition salts.

A preferred pharmaceutically acceptable salt of amlodipine is the besylate salt being the subject matter of US Patent 4,879,303; furthermore the maleate salt as set forth in US Patent 4,572,909; both patents are incorporated by reference herein in their entirety.

Preferably, the ACEI is benazepril or a salt thereof, most preferably the hydrochloride thereof. Suitable salts of benazepril and benazeprilat can be found in US Patent No. 4,410,520 and which is incorporated by reference herein in its entirety. For purposes of the present invention, the hydrochloride salt of the ACEI is most advantageous, with the most preferred specific ACEI compound being benazepril hydrochloride.

The most preferred active components according to the instant invention are amlodipine besylate and benazepril hydrochloride.

Preferred dosage ranges in combinations according to the instant invention comprise an amount of amlodipine or a pharmaceutically acceptable salt thereof from 21 mg to 40 mg, preferably from 6 mg to 40 mg and an amount of the ACEI or a pharmaceutically acceptable salt thereof from 81 mg to 160 mg, preferably from 20 mg to 160 mg, in each case, corresponding to the free base. Within such combinations the amount of amlodipine or a pharmaceutically acceptable salt thereof is preferably from 6 mg to 20 mg, especially 10 mg, in all above cases, corresponding to the free base. A preferred amount of benazepril or benazeprilat, respectively, or, in each case, pharmaceutically acceptable salt thereof is from 20 mg to 80 mg, preferably 20 mg to 40 mg, e.g., 20, 30, or 40 mgs, especially 20 mgs, or from 40 mg to 160 mg, especially 40 mg to 120 mg, most preferably 40 mg to 80 mg,. e.g., 40, 50, 60 70 or 80 mgs, esp. 40 mgs; in all above cases, corresponding to benazepril hydrochloride.

A preferred amount of amlodipine or a pharmaceutically acceptable salt thereof is 10 or 20 mg and preferred amounts of benazepril or a pharmaceutically acceptable salt thereof are 20 mg, 40 mg or 80 mg. Most preferably all said doses are daily doses. As used herein, a "daily dose" refers to the total amount of the drug substance administered in a 24 hour period, with a single administration the preferred method of treatment.

The active ingredients of the pharmaceutical composition according to the present invention as described herein may be used for simultaneous use or sequential use in any order, for separate use or most preferably as a fixed combination.

While the CCB and the ACEI can be administered at different times, they are most preferably administered at the same time. Most conveniently, this is via a single, fixed combination dosage form. However, the CCB can be administered at times different from the administration of the ACEI and the invention benefits still be realized. When administered at different times, the CCB and the ACEI should be given within about 16 hours of each other, preferably within about 12 hours of each other, more preferably within about 8 hours of each other, most preferably within about 4 hours of each other. Of course, these time periods can be extended if the dosage form is one that will "administer" the agents for extended periods.

When the CCB and the ACEI are given substantially simultaneously, they may be given by a single fixed combination dosage form or by different dosage forms, whichever are convenient. When given by different dosage forms, it is irrelevant whether the route of administration is the same for each agent or different for each agent. Any route of administration known for the individual agents is acceptable for the practice of the present invention. Most preferably, the agents are given in a fixed combination, or at least substantially simultaneously, i.e. within about 1 hour of each other. Also, the most suitable dosage form is an oral dosage form, where an oral administration is a clinically suitable route.

In a variation thereof, the present invention likewise relates to a "kit-of-parts", for example, in the sense that the components to be combined according to the present invention can be dosed independently or by use of different fixed combinations with distinguished amounts of the components, i.e. simultaneously or at different time points. The parts of the kit of parts can then e.g. be administered simultaneously or chronologically staggered, that is at different time points and with equal or different time intervals for any part of the kit of parts. Preferably, the time intervals are chosen such that the effect on the treated disease or condition in the combined use of the parts is more beneficial than the effect that would be obtained by use of only any one of the components.

Dosages of the two agents indude all dosages at which the agents are used individually. Corresponding dosages for other salts of amlodipine, for free benazepril and other salts of benazepril, and benazeprilat and its salts will be readily apparent to those of ordinary skill in the art. In each of the dosages set forth here, the range is the acceptable range based on adult mammal of approximately 50 to about 70 kg. Modified dosage ranges for mammals of other sizes and stages of development will be apparent to those of ordinary skill in the art.

Benazepril and amlodipine are normally physically incompatible substances. Hence, if incorporated into a single dosage form they must be kept physically separated. This may be accomplished in any of the myriad ways known in the art, such as bi-layered tablets, coated pellets of one agent incorporated into a tablet of the other, separately coated pellets of each agent in a capsule or tablet, coated pellets of one agent in capsule together with powder of the other agent, each agent microencapsulated separately and then blended together for use in a tablet or capsule, use of a dual or multiple compartment transdermal device, etc. Due to the incompatibility, combination products of the two agents in an injectable solution may not really be acceptable. For convenience purposes, a coated compressed tablet of benazepril together with amlodipine powder in a capsule has been found to be the most desirable oral form.

For example, a pharmaceutical formulation of the present invention comprises between about 20 mg and about 160 mg of benazepril hydrochloride; between about 3.4 mg and about 25.843 mg of lactose, monohydrate; between about 3.4 mg and about 6.801 mg of pregelatinized starch; between about .068 mg and about 1.360 mg of colloidal SiO₂; between about 1.360 mg and about 5.440 mg of crospovidone; between about 23.4 mg and about 185.843 mg of microcrystalline cellulose; between about 1.340 mg and about 8.4 mg of magnesium stearate; between about 6 mg and about 40 mg of amlodipine besylate; between about 20 mg and about 160 mg of calcium phosphate dibasic; and between about 2 mg and about 16 mg of sodium starch glycolate. Optionally added to the formulation is between about 1.5 mg and about 7.5 mg of hydroxypropyl methylcellulose; purified water; between about 0.075 mg and about 0.375 mg of polysorbate 80; and a trace of talc.

Preferably, the pharmaceutical formulation is a capsule formulation comprising a core comprising between about 20 mg and about 160 mg of benazepril hydrochloride; between about 3.4 mg and about 25.843 of lactose, monohydrate; between about 3.4 mg and about 6.801 mg of pregelatinized starch; between about 0.068 mg and about 1.360 mg of colloidal SiO₂; between about 1.360 mg and about 5.440 mg of crospovidone; between about 3.4 mg and about 25.843 mg of microcrystalline cellulose; between about .340 mg and about 3.4 mg of magnesium stearate; and a powder comprising between about 6 mg and about 40 mg of amlodipine besylate; between about 20 mg and about 160 mg of microcrystalline cellulose; between about 20 mg and about 160 mg of calcium phosphate dibasic; between about 2 mg and about 16 mg of sodium starch glycolate; and between about 1 mg and about 5 mg of magnesium stearate. Optionally added to the formulation is between about 1.5 mg and about 7.5 mg of hydroxypropyl methylcellulose; purified water, between about 0.075 mg and about 0.375 mg of polysorbate 80; and a trace of talc.

For the present purposes, preferred mammals are rabbits, dogs, goats, hogs, sheep; horses, cattle, and primates, more preferably primates, most preferably humans.

The invention furthermore relates to a commercial package comprising the combination according to the present invention together with instructions for simultaneous, separate or sequential use.

The following examples are presented to exemplify, but not to limit the invention.

### Example 1

One thousand capsules containing 20 mg of benazepril hydrochloride and amlodipine besylate equivalent to 5 mg of amlodipine base for use in the present invention were prepared as follows:
Benazepril hydrochloride cores are prepared using the following:

| | | |
|---|---|---|
| 1. | Benazepril HCL | 20.000 mg |
| 2. | Lactose, monohydrate | 32.920 mg |
| 3. | Pregetatinized Starch | 5.000 mg |
| 4. | Colloidal SiO₂ | 1.000 mg |
| 5. | Crospovidine | 2.000 mg |
| 6. | Microcrystalline Cellulose | 10.000 mg |
| 7. | Hydrogenated Castor Oil | 4.000 mg |
| 8. | Purified Water | as needed |

Components 1-3 are milled and blended together and water is added to granulate the blend. The wet granules are screened and oven dried. The dried granules are then milled together with components 5-7. Component 4 is screened and then mixed with the other ingredients. The resulting mixture is then compressed into a core.

The thus made cores are coated with a coating solution prepared as follows:

| | | |
|---|---|---|
| 9. | Hydroxypropyl Methylcellulose 2910,3cps | 4.881 mg |
| 10. | Polysorbate 80 | 0.119 mg |
| 11. | Purified Water | as needed |
| 12. | Talc | trace |

Component 10 is dissolved in the water and component 9 is added thereto. The previously made cores are then coated with this solution and the wet coated tablets are dried. The dried tablets are then dusted with component 12.

Amlodipine besylate for incorporation into the formulation is prepared as follows:

| | | |
|---|---|---|
| 13. | Amlodipine Besylate | 6.944 mg |
| 14. | Microcrystalline Cellulose | 124.056 mg |
| 15. | Calcium Phosphate Dibasic | 63.000 mg |
| 16. | Sodium Starch Glycolate | 4.000 mg |
| 17. | Magnesium Stearate | 2.000 g |

Components 13-16 are mixed together and the blended mixture is screened and reblended. Component 17 is separately screened and then blended with the reblended mixture containing the amlodipine.

No. 1 hard gelatin capsules are used to encapsulate one benazepril hydrochloride containing coated core along with 200 mg of the amlodipine besylate containing powder per capsule.

### Example 2

One thousand capsules containing 40 mg of benazepril hydrochloride and amlodipine besylate equivalent to 10 mg of amlodipine base for use in the present invention were prepared as follows:
Benazepril hydrochloride cores are prepared using the following:

| | | |
|---|---|---|
| 1. | Benazepril HCL | 40.000 mg |
| 2. | Lactose, monohydrate | 7.090 mg |
| 3. | Pregelatinized Starch | 5.440 mg |
| 4. | Colloidal SiO₂ | .907 mg |
| 5. | Crospovidine | 3.630 mg |
| 6. | Microcrystalline Cellulose | 9.070 mg |
| 7. | Magnesium Stearate | 1.870 mg |
| 8. | Purified Water | trace |

1. Premix in a suitable low shear type granulator, Benazepril drug substance, lactose monohydrate and pregelatinized starch for 20 minutes (range is about 5 to about 30 minutes).
2. Granulate premixed powders in with 26% purified water (range is about 16 to about 30 %).
3. After water addition, continue to granulate for 15 minutes (range is about 5 to about 30 min).
4. Mill the wet granulation through a suitable screening mill.
5. Dry wet granulation using suitable drying equipment, such as a fluidized bed dryer to less than 1% (range is about 0.5% to about 2%) of residual water (loss on drying).
6. Mill dried granulation through a suitable screening mill, together with colloidal silicon dioxide, crospovidone and microcrystalline cellulose.
7. Load milled granulation into suitable blending equipment
8. Add to the powders in the blender, magnesium stearate (screened through a suitable size screen), and blend for 5 min. (range is about 2 to about 10 min)
9. Compress blended material into tablet cores using a suitable rotary type compression machine.

The thus made cores are optionally coated with a coating solution prepared as follows:

| | | |
|---|---|---|
| 9. | Hydroxypropyl Methylcellulose 2910, 3cps | 4.881 mg |
| 10. | Polysorbate 80 | 0.119 mg |
| 11. | Purified Water | as needed |
| 12. | Talc | trace |

Component 10 is dissolved in the water and component 9 is added thereto. The previously made cores are then coated with this solution and the wet coated tablets are dried. The dried tablets are then dusted with component 12.

Amlodipine besylate for incorporation into the formulation is prepared as follows:

| | | |
|---|---|---|
| 13. | Amlodipine Besylate | 13.888 mg |
| 14. | Microcrystalline Cellulose | 117.112 mg |
| 15. | Calcium Phosphate Dibasic | 63.000 mg |
| 16. | Sodium Starch Glycolate | 4.000 mg |
| 17. | Magnesium Stearate | 2.000 mg |

Components 13-16 are mixed together and the blended mixture is screened and reblended. Component 17 is separately screened and then blended with the reblended mixture containing the amlodipine.

No. 1 hard gelatin capsules are used to encapsulate one benazepril hydrochloride containing coated core along with 200 mg of the amlodipine besylate containing powder (range about 196 mg to about 218 mg) per capsule.

## Claims

1. A Pharmaceutical composition comprising:
(1) an ACEI selected from the group consisting of benazepril, benazepnlat, and pharmaceutically acceptable salts thereof, and
(2) amlodipine or pharmaceutically acceptable salt thereof,
for the manufacture of a medicament for the treatment or prevention or delay of progression of a condition selected from the group consisting of hypertension, congestive heart failure, angina, myocardial infarction, atherosclerosis, diabetic nephropathy, diabetic cardiac myopathy, renal insufficiency, peripheral vascular disease, left ventricular hypertrophy, cognitive dysfunction, blood pressure-related cerebrovasular disease, stroke, pulmonary disease or pulmonary hypertension and headache;
wherein
(i) the amount of amlodipine or a pharmaceutically acceptable salt thereof is corresponds to 6 mg to 40 mg of the free base and
(ii) the amount of the ACE inhibitor or a pharmaceutically acceptable salt thereof corresponds to 20 mg to 160 mg of benazepril hydrochloride.

2. The pharmaceutical composition of claim 1 wherein amlodipine is amlodipine besylate.

3. The pharmaceutical composition of claim 1 wherein the benazepril is benazepril hydrochloride.

4. The pharmaceutical composition of daim 1 wherein the amount of amlodipine or a pharmaceutically acceptable salt thereof corresponds to from 6 mg to 20 mg of the free base.

5. The pharmaceutical composition of claim 1 wherein the amount of benazepril or a pharmaceutically acceptable salt thereof corresponds to 20 mg to 40 mg of benazepril hydrochloride.

6. The pharmaceutical composition of claim 1 wherein the amount of benazepril or a pharmaceutically acceptable salt thereof corresponds to from 40 mg to 80 mg of benazepril hydrochloride.

7. The pharmaceutical composition of claim 1 wherein the amlodipine or a pharmaceutically acceptable salt thereof and the benazepril or a pharmaceutically acceptable salt thereof are administered in a single dosage form, such that the amlodipine and the benazepril are physically separated from each other.

8. A method for the treatment or prevention or delay of progression of a condition selected from the group consisting of hypertension, congestive heart failure, angina, myocardial infarction, atherosclerosis, diabetic nephropathy, diabetic cardiac myopathy, renal insufficiency, peripheral vascular disease, left ventricular hypertrophy, cognitive dysfunction, blood pressure-related cerebrovasular disease, stroke pulmonary disease or pulmonary hypertension and headache comprising administering a therapeutically effective amount of
(1) an ACEI selected from the group consisting of benazepril, benazeprilat, and pharmaceutically acceptable salts thereof, and
(2) amlodipine or pharmaceutically acceptable salt thereof, and
(3) a pharmaceutically acceptable carrier;
wherein
(i) the amount of amlodipine or a pharmaceutically acceptable salt thereof corresponds to 6 mg to about 40 mg of the free base and
(ii) the amount of the ACE inhibitor or a pharmaceutically acceptable salt thereof corresponds to 20 mg to 160 mg of benazepril hydrochloride.

9. The method of claim 9 wherein the amlodipine is amlodipine besylate.

10. The method of claim 9 wherein the benazepril is benazepril hydrochloride.

11. The method of claim 9 wherein the amlodipine is amlodipine besylate and the benazepril is benazepril hydrochloride.

12. The method of daim 9 wherein the amount of amlodipine or a pharmaceutically acceptable salt thereof corresponds to from 6 mg to 20 mg of amlodipine free base; and
wherein the amount of benazepril or a pharmaceutically acceptable salt thereof is selected from an amount corresponding to 20 mg to 40 mg, from 40 mg to 80 mg, and from 80 mg to120 mg of benazepril hydrochloride.

13. The method of claim 9 for simultaneous use or sequential use in any order, for separate use or most preferably as a fixed combination.

14. The method of claim 9 wherein the amlodipine and the benazepril are administered in a single dosage form, such that the amlodipine and the benazepril are physically separated from each other.

15. The method of claim 9 wherein the single dosage form comprises a capsule comprising within it (a) amlodipine powder and (b) a coated compressed tablet of benazepril.

16. The method of claim 9 wherein the amlodipine or a pharmaceutically acceptable salt thereof is administered in a second formulation that is free of the benazepril or a pharmaceutically acceptable salt thereof and the benazepril is administered in a first formulation that is free of the amlodipine.

17. The method of claim 9 wherein said first formulation and said second formulation are administered within about one hour of each other.

18. The method of claim 9 wherein the amount of amlodipine or a pharmaceutically acceptable salt thereof and benazepril or a pharmaceutically acceptable salt thereof, in each case, is the daily dosage.

19. A commercial package comprising the composition of daim 1 together with instructions for simultaneous, separate or sequential use.

20. A pharmaceutical formulation comprising:
between about 20 mg and about 160 mg of benazepril hydrochloride;
between about 3.4 mg and about 25.843 mg of lactose, monohydrate;
between about 3.4 mg and about 6.801 mg of pregelatinized starch;
between about .068 mg and about 1.360 mg of colloidal SiO₂;
between about 1.360 mg and about 5.440 mg of crospovidone;
between about 23.4 mg and about 185.843 mg of microcrystalline cellulose;
between about 1.340 mg and about 8.4 mg of magnesium stearate;
between about 6 mg and about 40 mg of amlodipine besylate;
between about 20 mg and about 160 mg of calcium phosphate dibasic; and
between about 2 mg and about 16 mg of sodium starch glycolate.

21. The pharmaceutical formulation of claim 20 further comprising:
between about 1.5 mg and about 7.5 mg of hydroxypropyl methylcellulose; water;
between about 0.075 mg and about 0.375 mg of polysorbate 80; and
a trace of talc.

22. A capsule formulation comprising
a core comprising
between about 20 mg and about 160 mg of benazepril hydrochloride;
between about 3.4 mg and about 25.843 of lactose, monohydrate;
between about 3.4 mg and about 6.801 mg of pregelatinized starch;
between about 0.068 mg and about 1.360 mg of colloidal SiO₂; between about 1.360 mg and about 5.440 mg of crospovidone; between about 3.4 mg and about 25.843 mg of microcrystalline cellulose; between about .340 mg and about 3.4 mg of magnesium stearate; and
a powder comprising:
between about 6 mg and about 40 mg of amlodipine besylate;
between about 20 mg and about 160 mg of microcrystalline cellulose;
between about 20 mg and about 160 mg of calcium phosphate dibasic;
between about 2 mg and about 16 mg of sodium starch glycolate; and
between about 1 mg and about 5 mg of magnesium stearate.

23. The capsule formulation of claim 22 further comprising a coating solution comprising:
between about 1.5 mg and about 7.5 mg of hydroxypropyl methylcellulose;
water,
between about 0.075 mg and about 0.375 mg of polysorbate 80; and
a trace of talc.

24. A method of coating a core with the coating solution of daim 23 comprising:
dissolving the polysorbate 80 in the water and adding hyroxypropyl methylcellulose to provide a solution;
coating the core with the solution;
drying the coated core; and
dusting the dried core with talc.

25. A method of preparing a pharmaceutical formulation comprising:
premixing benazepril hydrochloride, lactose monohydrate and pregelatinized starch for about 5 minutes to about 30 minutes;
granulating premixed powder in from about 16% to about 30% water;
after water addition, granulating for from about 5 minutes to about 30 minutes;
milling the wet granulation;
drying wet granulation to less than about 0.5% to about 2% of residual water,
milling dried granulation together with colloidal silicon dioxide, crospovidone and microcrystalline cellulose;
blending together with magnesium stearate for from about 2 minutes to about 10 minutes;
compressing blended material into tablet cores; and
encapsulating the cores with amlodipine besylate.

26. The method of claim 25 further comprises coating the cores with a coating solution prior to encapsulating with amlodipine besylate.

27. The method of claim 26 wherein the coating solution comprises:
between about 1.5 mg and about 7.5 mg of hydroxypropyl methylcellulose; water;
between about 0.075 mg and about 0.375 mg of polysorbate 80; and
a trace of talc.

28. A method of coating a core with the coating solution of daim 27 comprising:
dissolving the polysortiate 80 in the water and adding hyroxypropyl methylcellulose to provide a solution;
coating the core with the solution;
drying the wet coated core; and
dusting the dried core with talc.
